# EUROPEAN PATENT SPECIFICATION

(11) **EP 1 381 861 B1**
(45) Date of publication and mention of the grant of the patent: **20.01.2010**
(21) Application number: 02753678.8
(22) Date of filing: 20.03.2002
(51) Int. Cl.: G01N 33/53, G01N 33/553, G01N 33/543, B01J 13/14

(54) **PROCESSES FOR PRODUCING COATED MAGNETIC MICROPARTICLES AND USES THEREOF**
VERFAHREN ZUR HERSTELLUNG BESCHICHTETER MAGNETISCHER MIKROPARTIKEL UND DEREN VERWENDUNGEN
PROCEDES DE PRODUCTION DE MICROPARTICULES MAGNETIQUES ENROBEES ET APPLICATIONS DE CELLES-CI

(30) Priority: 20.03.2001 CN 01109870
(43) Date of publication of application: 21.01.2004
(73) Proprietor: CapitalBio Corporation, Changping District Beijing 102206 (CN)
(72) Inventor: CHEN, Depu, Liudaokou Road, Beijing 100083 (CN); XIE, Xin, Huhhot, Inner Mongolia Province 010010 (CN); ZHANG, Xu, Teachers College of Prof. Tehcnology, Ya An, Sichuan Province 625014 (CN); SUN, Baoquan, Linyi, Shangdong Province 276023 (CN); ZHOU, Yuxiang, Hai Dian District., Beijing 100084 (CN); FEI, Weiyang, Hai Dian District, Beijing 100084 (CN); CHENG, Jing, Beijing 100084 (CN)
(74) Representative: Vossius & Partner
(86) International application number: PCT/US2002/008798
(87) International publication number: WO 2002/075309

(56) References cited:
- EP-A- 0 234 083
- DE-A1- 19 800 294
- DE-A1- 19 800 894
- US-A- 4 335 094
- US-A- 4 452 773
- US-A- 4 454 234
- US-A- 4 620 987
- US-A- 4 873 102
- US-A- 4 891 324
- US-A- 4 929 400
- US-A- 5 076 950
- US-A- 5 091 206
- US-A- 6 048 920
- XIN XIE: "Preparation and application of surface-coated superparamagnetic nanobeads in the isolation of genomic DNA" J. OF MAGN. AND MAGN. MATER., vol. 227, 2004, pages 16-23,

## Description

### Technical Field

This invention relates generally to the field of production of coated magnetizable microparticles and uses thereof In particular, the invention provides a process for producing coated magnetizable microparticles with active functional groups, which process comprises, *inter alia,* conducting polymerization of coating monomers on the surface of magnetic microparticles to form coated magnetizable microparticles with active functional groups in the presence of a coupling agent, coating monomers, a functionalization reagent, a cross-linking agent and an initiator in an organic solvent containing a surfactant The coated magnetizable microparticles produced according to the present processes and uses of the coated magnetizable microparticles in a method for isolating and manipulating various moieties are also provided.

### Background Art

It is important and often crucial to separate, purify and test proteins (such as various antigens, antibodies and enzymes), nucleic acids and cells from a sample in biology, clinical medicine and many other fields. The separation and purification of biological materials can sometimes take up to 90% of time and cost of a procedure or task. Present separation and purification methods include precipitation, centrifugation, and chromatography to separate genomic DNA. However, some of them need expensive instruments; some of them have poor separation and purification results in spite of simple operations; and some of them are complicated and time-consuming. The current demands for sample preparation procedures include: automation and miniaturization, not using or using poisonous or toxic reagents as little as possible, rapid and efficient process, resulting prepared products suitable for subsequent manipulation, *e*.*g*., biochemical manipulation; and cost effectiveness.

In recent decades, magnetic particles have been used in the purification and separation of biological materials. As a relatively new type of functional polymeric materials, magnetic micro-beads have great potential applications in biomedicine (clinical diagnosis, immunization analysis, enzyme label, target drug, etc.), cytology (cell labels, cell separation, etc.), molecular biology (cDNA library, gene sequencing, extraction and hybridization of DNA and mRNA, etc.), biological engineering and burgeoning bio-chip technology. Compared with conventional separation methods, the novel separation technology using magnetic micro-beads does not need large expensive instruments, requires simple and rapid experimental procedure and has high separation efficiency. Up to now the commonly used magnetic beads in biology are micron-sized particles that are mainly used in cell separation. For example, the magnetic micro-beads bound with lectin can be used to separate T cells in medulla. However, studies on the magnetic nano-beads and their direct coating and functionalization are still relatively rare. The separation technique using magnetic nano-beads has wide application potential. Because of the excellent soft magnetic characteristics, magnetic nano-beads can be used as targeted therapeutic agents, *e*.*g*., can be guided to a desired pathological locus by a magnetic force and effect their therapeutic effect via a therapeutic agent carried by the magnetic nano-beads.

The magnetic micro-beads coated with functional groups comprise inner magnetic micro-crystals and surface coating polymer. Certain preparation methods for coated and functionalized magnetic micro-beads are known. One such known method is to prepare magnetic iron oxide particles first and then coat the prepared magnetic iron oxide particles. However, the polarity of the inner magnetic microcrystals is strong and it is difficult to coat the inner magnetic microcrystals with a polymer layer having a weak polarity. During the coating process, the monomer can polymerize in solution instead of on the surface of the magnetic micro-beads. The nano-particles may also aggregate with each other during the coating process. Current coating methods include physical methods and chemical methods. The embedding method is commonly used in physical coating. In the embedding process, the magnetic micro-beads are dispersed in a polymer solution. Then solvent is removed through pulverization, flocculation, aggradation and evaporation to obtain the polymer-coated magnetic micro-beads. However, the binding between the inner magnetic microcrystals and the outer layer of the coated magnetic micro-beads, mainly via Van der Waals force, is weak and the polymer layer can be broken off easily. The coated microbeads have wide diameter distribution and irregular shapes. For example, magnetic crystals can be dispersed directly in amiaodextron, polymethacrylic acid or the lecithin solution. Then the magnetic crystals are coated with polymer through physical adsorption. This method can not be used to coat magnetic micro-beads smaller than 100 nanometers because of particle aggregation. The suspension-polymerization is commonly used in the chemical method. In this process, the monomers polymerize on the surface of the magnetic micro-beads. Silanization of the magnetic micro-beads' surface is mainly used to make the polymerization on the surface viable. But the coating process must be performed step by step and is complicated. And in the process, HCl gas is produced and will corrupt the micro-beads. Because the diameter of the nano-crystals is small and surface energy is high, the nano-crystals will be easily corrupted. As a result, the surface can be broken and the diameter can be changed. So the silanization of the surface is not suitable for coating and functionalization of the nano-crystals.

US 4,454,234 A discloses coated magnetizable particles of sub-micron size used to form reversible suspensions in which the particles may be magnetically aggregated and thereafter resuspended as desired.

Xu et al., Applied Surface Science 120 (1997) 269-278 disclose the direct silanation of nanosized superparamagnetic particles (γ-Fe₂O₃) using 3-aminopropyl triethoxy silane, wherein the silanation is conducted in both organic (toluene) and water solutions to examine the solvent effect on the molecular orientation and packing density of the silanized films. In acidic environment, the films silanized in toluene are more stable than that in water, but both are unstable in basic environment.

There exists a need in the art for a new process for producing coated magnetizable microparticles. This invention address this and other related needs in the art.

### Disclosure of the Invention

In one aspect, the present invention is directed to a process for producing coated magnetizable microparticles with active functional groups, which process comprises: a) dispersing magnetizable microparticles with a diameter ranging from 5 to 1,000 nanometers in an organic solvent containing a surfactant; b) adding a coupling agent, coating monomers, a functionalization reagent, a cross-linking agent and an initiator to said organic solvent containing said dispersed magnetizable microparticles, allowing attachment of said coupling agent to the surface of said magnetizable microparticles and dispersing said coating monomers evenly on the surface of said magnetizable microparticles with said attached coupling agent; c) initiating and completing polymerization of said coating monomers to form coated magnetizable microparticles with active functional groups in the absence of oxygen, whereby polymers of said coating monomers are attached to the surface of said coated magnetizable microparticles via said coupling agent, multiple said polymers are crosslinked together via said cross-linking agent and said functionalization reagent is linked to said polymers, cross-linking agent and/or coupling agent so that at least one functional group of said functionalization reagent remains available, wherein the coupling reagent is selected from the group consisting of bis (2-hydroxyethyl methacrylate) phosphate, bis (trimethylolpropane diocrylate) phospate, and bis (pentaerythritol triacrylate) phosphate.

In another aspect, the present invention is directed to coated magnetizable microparticles with active functional groups, which are obtainable according to the above process.

In still another aspect, the present invention is directed to a method for isolating a moiety, which method comprises: a) providing coated magnetizable microparticles, which are obtained according to the above process, comprising a binding partner that is capable of binding to a moiety to be isolated; b) contacting a sample containing or suspected of containing said moiety with said coated magnetizable microparticles provided in step a) under conditions allowing binding between said moiety and said binding partner; and c) recovering said coated magnetizable microparticles from said sample with a magnetic force.

In yet another aspect, the present invention is directed to a method for manipulating a moiety, which method comprises: a) providing coated magnetizable microparticles, which are obtained according to the above process, comprising a binding partner that is capable of binding to a moiety to be manipulated; b) coupling said moiety to said coated magnetizable microparticles provided in step a) via binding between said moiety and said binding partner to form a moiety-coated-magnetizable-microparticles complex; and c) manipulating said moiety-coated-magnetizable-microparticles complex with a magnetic force, thereby said moiety is manipulated.

### Brief Description of the Drawings

Figure 1 illustrates the magnetization hysteresis loops of coated Fe₃O₄ magnetic nanoparticles as described in example 1.
Figure 2 illustrates the FT-IR spectra of coated Fe₃O₄ magnetic nanoparticles as described in example 1.
Figure 3 illustrates, in a magnified view of the enrichment of the coated magnetizable microparticles when the electromagnetic pole is electrified as described in example 6.

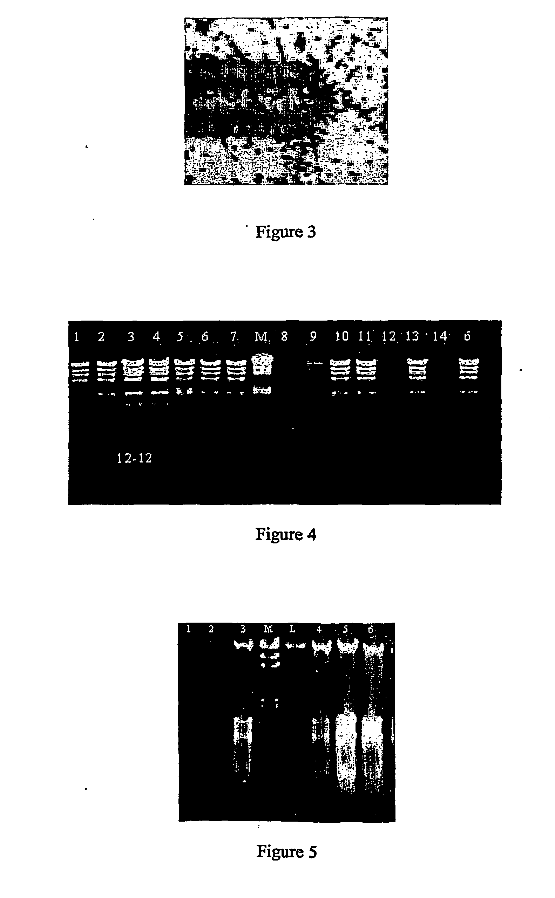

Figure 4 illustrates an exemplary agarose gel electrophoresis of genomic DNA isolated using the present coated magnetizable microparticles in different ionic, pH and solvent conditions as described in example 8. Lanes 1: 100 µl NaI + 100 µl isopropanol; **2**: 50 µl NaI + 150 µl isopropanol; **3**: 150 µl NaI + 50 µl isopropanol; **4**: 100 µl NaI + 100 µl ethanol; **5**: 100 µl NaCl + 100 µl ethanol; **6**: NaI + 200 µl PEG; **7**: NaI + 200 µl PEG + 100 µl ethanol; **8**: 100 µl NaCl + 100 µl isopropanol; **9**: 100 µl NaI + 100 µl methanol; **10**: NaI + 100 µl urea + 100 µl isopropanol; **11**:100 µl SDS + 100 µl ethanol; **12**: 100 µl SDS + 100 µl methanol; **13**: NaI + 100 µl urea + 100 µl ethanol; **14**: NaI + 100 µl urea + 100 µl methanol; and M: λDNA markers (Hind III single digestion).
Figure 5 illustrates an exemplary agarose gel electrophoresis of genomic DNA isolated from *E.coli* using different coated magnetizable microparticles as described in example 9. Lanes **1:** uncoated magnetizable microparticles; **2:** uncoated magnetizable microparticles; **3:** magnetizable microparticles coated with polystyrene; **4:** magnetizable microparticles coated with methyl methacrylate; **5:** magnetizable microparticles coated with glucosamine; **6:** magnetizable microparticles coated with methacrylic acid; L: phenolchloroform isolated DNA; and M: λDNA markers (Hind III single digestion).

### Modes of Carrying Out the Invention

For clarity of disclosure, and not by way of limitation, the detailed description of the invention is divided into the subsections that follow.

### A. Definitions

Unless defined otherwise, all technical and scientific terms used herein have the same meaning as is commonly understood by one of ordinary skill in the art to which this invention belongs. All patents, applications, published applications and other publications referred to herein are incorporated by reference in their entirety. If a definition set forth in this section is contrary to or otherwise inconsistent with a definition set forth in the patents, applications, published applications and other publications that are herein incorporated by reference, the definition set forth in this section prevails over the definition that is incorporated herein by reference.

As used herein, "a" or "an" means "at least one" or "one or more".

As used herein, "magnetic substance" refers to any substance that has the properties of a magnet, pertaining to a magnet or to magnetism, producing, caused by, or operating by means of, magnetism.

As used herein, "magnetizable substance" refers to any substance that has the property of being interacted with the field of a magnet, and hence, when suspended or placed freely in a magnetic field, of inducing magnetization and producing a magnetic moment. Examples of magnetizable substance include, but are not limited to, paramagnetic, ferromagnetic and ferrimagnetic substances. "Magnetizable microparticles" refers to any microparticles that comprise a magnetizable substance. "Magnetic microparticles" include magnetizable microparticles and microparticles having permanent magnetic property.

As used herein, "paramagnetic substance" refers to the substances where the individual atoms, ions or molecules possess a permanent magnetic dipole moment. In the absence of an external magnetic field, the atomic dipoles point in random directions and there is no resultant magnetization of the substances as a whole in any direction. This random orientation is the result of thermal agitation within the substance. When an external magnetic field is applied, the atomic dipoles tend to orient themselves parallel to the field, since this is the state of lower energy than antiparallel position. This gives a net magnetization parallel to the field and a positive contribution to the susceptibility. Further details on "paramagnetic substance" or "paramagnetism" can be found in various literatures, *e*.*g*., at page 169 - pare 171, Chapter 6, in "Electricity and Magnetism" by B.I. Bleaney and B. Bleaney, Oxford, 1975.

As used herein, "ferromagnetic substance" refers to the substances that are distinguished by very large (positive) values of susceptibility, and are dependent on the applied magnetic field strength. In addition, ferromagnetic substances may possess a magnetic moment even in the absence of the applied magnetic field, and the retention of magnetization in zero field is known as "remanence". Further details on "ferromagnetic substance" or "ferromagnetism" can be found in various literatures, *e*.*g*., at page 171 - page 174, Chapter 6, in "Electricity and Magnetism" by B.I. Bleaney and B. Bleaney, Oxford, 1975.

As used herein, "ferrimagnetic substance" refers to the substances that show spontaneous magnetization, remanence, and other properties similar to ordinary ferromagnetic materials, but the spontaneous moment does not correspond to the value expected for full parallel alignment of the (magnetic) dipoles in the substance. Further details on "ferrimagnetic substance" or "ferrimagnetism" can be found in various literatures, *e*.*g*., at page 519- 524, Chapter 16, in "Electricity and Magnetism" by B.I. Bleaney and B. Bleaney, Oxford, 1975.

As used herein, "metal oxide particle" refers to any oxide of a metal in a particle form. Certain metal oxide particles have paramagnetic or super-paramagnetic properties. "Paramagnetic particle" is defined as a particle which is susceptible to the application of external magnetic fields, yet is unable to maintain a permanent magnetic domain. In other words, "paramagnetic particle" may also be defined as a particle that is made from or made of "paramagnetic substances". Non-limiting examples ofparamagnetic particles include certain metal oxide particles, *e*.*g*., Fe₃O₄ particles, metal alloy particles, *e*.*g*., CoTaZr particles.

As used herein, "organic solvent" refers to a liquid organic compound with the power to dissolve or disperse solids, gases, or liquids (miscibility).

As used herein, "surfactant (or surface-active agent)" refers to a soluble compound that reduces the surface tension of liquids, or reduces interfacial tension between two liquids or a liquid and a solid.

As used herein, "coupling agent" refers to a compound having multiple ends wherein at least one end of the compound has high affinity to the magnetizable substance of the microparticles and at least another end of the compound has a double bond that can participate in the polymerization and anchor the polymerized coating monomers, functionalization reagent and cross-linking agent on the surface of the magnetizable microparticles.

As used herein, "coating monomers" refer to molecules that are capable of polymerizing with a number of the same, like or unlike molecules to form a polymer backbone. Preferably, each monomer has at least one double bond that is reactive with the double bond of the cross-linking agent, the functionalization reagent and/or the cross-linking agent

As used herein, "functionalization reagent" refers to a compound having multiple ends wherein at least one end of the compound has a double bond that can participate in the polymerization and at least another end of the compound has an active functional group that remains available after the polymerization. Exemplary active functional groups include, although limited to, carboxyl, amino, hydroxyl, sulfhydryl, hydrosulfuryl, epoxy, ester, alkene, alkyne, alkyl, aromatic, aldehyde, ketone, sulfate, amide, urethane group(s), or their derivatives thereof.

As used herein, "cross-linking agent" refers to a compound that is capable of crosslinking individual strands of a polymerized polymer chain. Preferably, the cross-linking agent has at least two double bonds that are reactive with the double bond of the coating monomers.

As used herein, "initiator" refers to a compound that, upon suitable stimulation, *e*.*g*., heating, is capable of generating free radicals to initiate the polymerization process among the coupling agent, coating monomers, functionalization reagent and/or cross-linking agent.

As used herein, "moiety" refers to any substance whose isolation or manipulation using the present coated magnetizable microparticles is desirable. Normally, the dimension (or the characteristic dimensions) of the moiety should not exceed 1 cm. For example, if the moiety is spherical or approximately spherical, the dimension of the moiety refers to the diameter of the sphere or an approximated sphere for the moiety. If the moiety is cubical or approximately cubical, then the dimension of the moiety refers to the side width of the cube or an approximated cube for the moiety. If the moiety has an irregular shape, the dimension of the moiety may refer to the average between its largest axis and smallest axis. Non-limiting examples of moieties include cells, cellular organelles, viruses, particles, molecules, *e*.*g*., proteins, DNAs and RNAs, or an aggregate or complex thereof.

As used herein, "binding partner" refers to any substance that binds to the moieties with desired affinity or specificity. Non-limiting examples of the binding partners include cells, cellular organelles, viruses, particles, microparticles or an aggregate or complex thereof, or an aggregate or complex of molecules, or specific molecules such as antibodies, single stranded DNAs. The binding partner can be a substance that is coated on the surface of the present coated magnetizable microparticles. Alternatively, the binding partner can be a substance that is incorporated, *e*.*g*., microfabricated, into the material composition of the present coated magnetizable microparticles. The material composition of the present coated magnetizable microparticles may possess binding affinity to certain moiety, and thus functioning as a binding partner itself.

As used herein, "chip" refers to a solid substrate with a plurality of one-, two- or three-dimensional micro structures or micro-scale structures on which certain processes, such as physical, chemical, biological, biophysical or biochemical processes, etc., can be carried out. The micro structures or micro-scale structures such as channels and wells, electrode elements, electromagnetic elements, are incorporated into, fabricated on or otherwise attached to the substrate for facilitating physical, biophysical, biological, biochemical, chemical reactions or processes on the chip. The chip may be thin in one dimension and may have various shapes in other dimensions, for example, a rectangle, a circle, an ellipse, or other irregular shapes. The size of the major surface of chips used in the present invention can vary considerably, *e*.*g*., from about 1 mm² to about 0.25 m². Preferably, the size of the chips is from about 4 mm² to about 25 cm² with a characteristic dimension from about 1 mm to about 7.5 cm. The chip surfaces may be flat, or not flat. The chips with non-flat surfaces may include channels or wells fabricated on the surfaces. One example of a chip is a solid substrate onto which multiple types of DNA molecules or protein molecules or cells are immobilized.

As used herein, "medium (or media)" refers to a fluidic carrier, *e*.*g*., liquid or gas, wherein a moiety, alone or bound to a magnetizable particle, is dissolved, suspended or contained.

As used herein, "microfluidic application" refers to the use of microscale devices, *e*.*g*., the characteristic dimension of basic structural elements is in the range between less than 1 micron to 1 cm scale, for manipulation and process in a fluid-based setting, typically for performing specific biological, biochemical or chemical reactions and procedures. The specific areas include, but are not limited to, biochips, *i.e*., chips for biologically related reactions and processes, chemchips, *i*.*e*., chips for chemical reactions, or a combination thereof The characteristic dimensions of the basic elements refer to the single dimension sizes. For example, for the microscale devices having circular shape structures (*e*.*g*. round electrode pads), the characteristic dimension refers to the diameter of the round electrodes. For the devices having thin, rectangular lines as basic structures, the characteristic dimensions may refer to the width or length of these lines.

As used herein, "micro-scale structures" mean that the structures have characteristic dimension of basic structural elements in the range from about 1 micron to about 20 mm scale.

As used herein, "plant" refers to any of various photosynthetic, eucaryotic multi-cellular organisms of the kingdom Plantae, characteristically producing embryos, containing chloroplasts, having cellulose cell walls and lacking locomotion.

As used herein, "animal" refers to a multi-cellular organism of the kingdom of Animalia, characterized by a capacity for locomotion, nonphotosynthetic metabolism, pronounced response to stimuli, restricted growth and fixed bodily structure. Non-limiting examples of animals include birds such as chickens, vertebrates such as fish and mammals such as mice, rats, rabbits, cats, dogs, pigs, cows, ox, sheep, goats, horses, monkeys and other non-human primates.

As used herein, "bacteria" refers to small prokaryotic organisms (linear dimensions of around 1 micron) with non-compartmentalized circular DNA and ribosomes of about 70S. Bacteria protein synthesis differs from that of eukaryotes. Many anti-bacterial antibiotics interfere with bacteria proteins synthesis but do not affect the infected host.

As used herein, "eubacteria" refers to a major subdivision of the bacteria except the archaebacteria. Most Gram-positive bacteria, cyanobacteria, mycoplasmas, enterobacteria, pseudomonas and chloroplasts are eubacteria. The cytoplasmic membrane of eubacteria contains ester-linked lipids; there is peptidoglycan in the cell wall (if present); and no introns have been discovered in eubacteria.

As used herein, "archaebacteria" refers to a major subdivision of the bacteria except the eubacteria. There are three main orders of archaebacteria: extreme halophiles, methanogens and sulphur-dependent extreme thermophiles. Archaebacteria differs from eubacteria in ribosomal structure, the possession (in some case) of introns, and other features including membrane composition.

As used herein, "virus" refers to an obligate intracellular parasite of living but non-cellular nature, consisting of DNA or RNA and a protein coat. Viruses range in diameter from about 20 to about 300 nm. Class I viruses (Baltimore classification) have a double-stranded DNA as their genome; Class II viruses have a single-stranded DNA as their genome; Class III viruses have a double-stranded RNA as their genome; Class IV viruses have a positive single-stranded RNA as their genome, the genome itself acting as mRNA; Class V viruses have a negative single-stranded RNA as their genome used as a template for mRNA synthesis; and Class VI viruses have a positive single-stranded RNA genome but with a DNA intermediate not only in replication but also in mRNA synthesis. The majority of viruses are recognized by the diseases they cause in plants, animals and prokaryotes. Viruses of prokaryotes are known as bacteriophages.

As used herein, "fungus" refers to a division of eucaryotic organisms that grow in irregular masses, without roots, stems, or leaves, and are devoid of chlorophyll or other pigments capable of photosynthesis. Each organism (thallus) is unicellular to filamentous, and possesses branched somatic structures (hyphae) surrounded by cell walls containing glucan or chitin or both, and containing true nuclei.

As used herein, "microparticles" refer to particles of any shape, any composition, any complex structures that can be used in the present coating processes and moiety isolation and manipulation methods. One example of microparticles are magnetic beads that are manipulable by magnetic forces. The microparticles used in the present processes and methods have a diameter ranging from 5 to 1,000 nanometers.

As used herein, "physical force" refers to any force that moves the moieties or their binding magnetizable particles without chemically or biologically reacting with the moieties and the magnetizable particles, or with minimal chemical or biological reactions with the magnetizable particles and the moieties so that the biological/chemical functions/properties of the magnetic particles and the moieties are not substantially altered as a result of such reactions. Throughout the application, the term of "forces" or "physical forces" always means the "forces" or "physical forces" exerted on a moiety or moieties, the binding partner(s) and/or the magnetizable particle(s). The "forces" or "physical forces" are always generated through "fields" or "physical fields". The forces exerted on moieties, the binding partner(s) and/or the magnetizable particles(s) by the fields depend on the properties of the moieties, the binding partner(s) and/or the magnetizable particles(s). Thus, for a given field or physical field to exert physical forces on a moiety, it is necessary for the moiety to have certain properties. While certain types of fields may be able to exert forces on different types of moieties having different properties, other types of fields may be able to exert forces on only limited type of moieties. For example, magnetic field can exert forces or magnetic forces only on magnetizable particles or moieties having certain magnetic properties, but not on other particles, *e*.*g*., polystyrene microdevices. On the other hand, a non-uniform electric field can exert physical forces on many types of moieties such as polystyrene microdevices, cells, and also magnetizable particles. It is not necessary for the physical field to be able to exert forces on different types of moieties or different moieties. But it is necessary for the physical field to be able to exert forces on at least one type of moiety or at least one moiety, the binding partner(s) and/or the magnetizable particle(s).

As used here in, "electric forces (or electrical forces)" are the forces exerted on moieties, the binding partner(s) and/or the magnetizable particle(s) by an electric (or electrical) field.

As used herein, "magnetic forces" are the forces exerted on moieties, the binding partner(s) and/or the magnetizable particle(s) by a magnetic field.

As used herein, "sample" refers to anything which may contain a moiety to be isolated or manipulated using the present coated magnetizable microparticles and/or methods. The sample may be a biological sample, such as a biological fluid or a biological tissue. Examples of biological fluids include urine, blood, plasma, serum, saliva, semen, stool, sputum, cerebral spinal fluid, tears, mucus, amniotic fluid or the like. Biological tissues are aggregates of cells, usually of a particular kind together with their intercellular substance that form one of the structural materials of a human, animal, plant, bacterial, fungal or viral structure, including connective, epithelium, muscle and nerve tissues. Examples of biological tissues also include organs, tumors, lymph nodes, arteries and individual cell(s). Biological tissues may be processed to obtain cell suspension samples. The sample may also be a mixture oftarget analyte or enzyme containing molecules prepared *in vitro.* The sample may also be a cultured cell suspension. In case of the biological samples, the sample may be crude samples or processed samples that are obtained after various processing or preparation on the original samples. For example, various cell separation methods (*e*.*g*., magnetically activated cell sorting) may be applied to separate or enrich target cells from a body fluid sample such as blood. Samples used for the present invention include such target-cell enriched cell preparation.

As used herein, a "liquid (fluid) sample" refers to a sample that naturally exists as a liquid or fluid, *e*.*g*., a biological fluid A "liquid sample" also refers to a sample that naturally exists in a non-liquid status, *e*.*g*., solid or gas, but is prepared as a liquid, fluid, solution or suspension containing the solid or gas sample material. For example, a liquid sample can encompass a liquid, fluid, solution or suspension containing a biological tissue.

As used herein the term "assessing (or assessed)" is intended to include quantitative and qualitative determination of the identity and/or quantity of a moiety, *e*.*g*., a protein or nucleic acid, present in the sample or on the present coated magnetizable microparticles or in whatever form or state. Assessment would involve obtaining an index, ratio, percentage, visual or other value indicative of the identity of a moiety in the sample and may further involve obtaining a number, an index, or other value indicative of the amount or quantity or the concentration of a moiety present in the sample or on the magnetizable particle or in whatever form or state. Assessment may be direct or indirect. Assessment may be qualitative or quantitative.

### B. Processes for producing coated magnetizable microparticles

In one aspect, the present invention is directed to a process for producing coated magnetizable microparticles with active functional groups, which process comprises: a) dispersing magnetizable microparticles with a diameter ranging from 5 to 1,000 nanometers in an organic solvent containing a surfactant; b) adding a coupling agent, coating monomers, a functionalization reagent, a cross-linking agent and an initiator to said organic solvent containing said dispersed magnetizable microparticles, allowing attachment of said coupling agent to the surface of said magnetizable microparticles and dispersing said coating monomers evenly on the surface of said magnetizable microparticles with said attached coupling agent; c) initiating and completing polymerization of said coating monomers to form coated magnetizable microparticles with active functional groups in the absence of oxygen, whereby polymers of said coating monomers are attached to the surface of said coated magnetizable microparticles via said coupling agent, multiple said polymers are crosslinked together via said cross-linking agent and said functionalization reagent is linked to said polymers, cross-linking agent and/or coupling agent so that at least one functional group of said functionalization reagent remains available, wherein the coupling reagent is selected from the group consisting of bis (2-hydroxyethyl methacrylate) phosphate, bis (trimethylolpropane diocrylate) phospate, and bis (pentaerythritol triacrylate) phosphate.

Any suitable magnetizable substance can be used in the present processes. Non-limiting examples of the magnetizable substances include ferrimagnetic substances, ferromagnetic substances, paramagnetic substances or superparamagnetic substances. In a specific embodiment, the present processes use a paramagnetic substance, *e*.*g*., a paramagnetic metal oxide composition. Preferably, the paramagnetic metal oxide composition is a transition metal oxide or an alloy thereof. Any suitable transition metals can be used, such as iron, nickel, copper, cobalt, manganese, tantalum (Ta), zinc and zirconium (Zr). In a preferred embodiment, the metal oxide composition is Fe₃O₄ or Fe₂O₃. In another example, the magnetizable substance used in the processes of the present invention comprises a metal composition. Preferably, the metal composition is a transition metal composition or an alloy thereof such as iron, nickel, copper, cobalt, manganese, tantalum, zirconium and cobalt-tantalum-zirconium (CoTaZr) alloy.

Any suitable organic solvent can be used in the present processes. For example, toluene, dimethylbenzene, tetrahydrofuran and ethanol can be used as the organic solvents in the present processes. Ethanol can be used alone or in a mixture with water. Any suitable surfactant can be used in the present processes. For example, an anionic surfactant, *e*.*g*., dodecyl sulfonic acid sodium salt or sodium dodecyl benzene sulfonate, a nonionic surfactant, *e*.*g*., alkylphenolpolyoxyethene ether, or a cationic surfactant, *e*.*g*., hexadecyltrimethylammonium bromide, can be used in the present processes. The surfactant can be used at any suitable concentration in the organic solvent. In one example, the concentration of the surfactant in the organic solution ranges from about 0.1 % (v/v) to about 5% (v/v). The magnetizable microparticles can be dispersed in an organic solvent by any suitable methods. For example, the magnetizable microparticles can be dispersed in an organic solvent containing a surfactant under ultra-sonication and/or stirring, *e*.*g*., magnetic stirring.

Bis(2-hydroxyethyl methacrylate) phosphate, bis(trimethylolpropane diacrylate) phosphate, and bis(pentaerythritol triacrylate) phosphate are used as a coupling reagent in the present processes.

Any suitable coating monomers can be used in the present processes. For example, acrylic acid, methacrylic acid, methyl methacrylate, 2-hydroxyethyl methacrylate, glycoldimethylacrylate, ethylene glycol diacrylate, ethylene glycol dimethacrylate, diethylene glycol methacrylate, diethylene glycol acrylate, diethylene glycol dimethacrylate, diethylene glycol diacrylate, diethylene glycol methacrylate, diethylene glycol acrylate, triethylene glycol dimethacrylate, trimethylolpropane trimethacrylate, pentaerythritol triacrylate, styrene, divinylbenzene and a mixture thereof can be used as coating monomers in the present processes.

Any suitable functionalization reagent can be used in the present processes. For example, acrylic acid, methacrylic acid, glycidyl acrylate, pentaerythritol diacrylate and methacrolein can be used as functionalization reagent in the present processes.

Any suitable cross-linking agent can be used in the present processes. For example, diethylene glycol dimethacrylate, diethylene glycol diacrylate, ethylene glycol diacrylate, ethylene glycol dimethacrylate, trimethylopropane trimethacrylate, pentaerythritol triacrylate and divinylbenzene can be used as cross-linking agent in the present processes.

Any suitable initiator can be used in the present processes. For example, benzoyl peroxide or 2,2'-azobisisobutyronitrile can be used as the initiator in the present processes.

The ratio between the sum of the coupling agent, the coating monomers, the functionalization reagent and the cross-linking agent versus the magnetizable microparticles can be in any suitable range depending on a number of factors such as the particular reagents used and the polymerization conditions. In one example, the ratio between the sum of the coupling agent, the coating monomers, the functionalization reagent and the cross-linking agent versus the magnetizable microparticles ranges from about 1:400 (w/w) to about 1:1 (w/w). Similarly, the percentages of coating monomers, coupling agent, cross-linking agent, functionalization reagent and initiator in the sum of these substances can be in any suitable range depending on a number of factors such as the particular reagents used and the polymerization conditions. For example, the percentages of coating monomers, coupling agent, cross-linking agent, functionalization reagent and initiator in the sum of the substances are: from about 0% (v/v) to about 80% (v/v) coating monomers, from about 1% (v/v) to about 10% (v/v) coupling agent, from about 10% (v/v) to about 80% (v/v) cross-linking agent, from about 5% (v/v) to about 40% (v/v) functionalization reagent and from about 1% (v/v) to about 5% (w/v) initiator.

The coating monomers can be dispersed on the surface of the magnetizable microparticles using any suitable methods. For example, the coating monomers can be evenly dispersed on the surface of the magnetizable microparticles and the attached coupling agent under stirring. Preferably, the stirring lasts at least 30 minutes.

The polymerization process should be conducted in the absence of oxygen. The absence of oxygen can be performed by any suitable methods. For example, the absence of oxygen can be performed via purging air with an inert gas, *e*.*g*., nitrogen, helium or argon.

The polymerization can be initiated by any suitable methods to generate free radicals from the initiator. In one example, the polymerization can be initiated by heating the initiator to release free radicals. The reaction mixture can be heated at any suitable temperatures to initiate the polymerization depending on the reagents used in the polymerization and other reaction parameters. In one specific embodiment, the initiator is heated to a temperature ranging from about 25°C to about 150°C. Preferably, the initiator is heated to about 80°C.

If stirring is used to disperse the coating monomers evenly on the surface of the magnetizable microparticles and the attached coupling agent, the stirring magnitude can be lowered prior to the initiation of the polymerization. For example, the stirring magnitude can be lowered to 30 rpm or less than 30 rpm. The polymerization is allowed to proceed for a sufficient amount of time, *e*.*g*., at least 2 hours, to ensure the completion of the polymerization.

The present process can further comprise removing non-magnetizable microparticles from the reaction mixture. The non-magnetizable microparticles can be removed from the reaction mixture by any suitable methods. In one example, the non-magnetizable microparticles are removed from the reaction mixture by depositing magnetizable microparticles under magnetic stirring and removing supernatant. The process can also further comprise removing non-polymerized substances from the magnetizable microparticles. The non-polymerized substances can be removed from the magnetizable microparticles by any suitable methods. For example, the non-polymerized substances can be removed from the magnetizable microparticles via washing and filtration. Preferably, the non-polymerized substances are removed from the magnetizable microparticles via washing the magnetizable microparticles with deionized water and acetone.

The present process can further comprise incorporating a binding partner that is capable of binding, preferably specifically binding, to a moiety, in the coated magnetizable microparticles. The binding partner can be incorporated into the magnetizable microparticles prior to, concurrently with and subsequent to the polymerization. Any suitable binding partners can be used. Exemplary binding partners include a cell, cellular organelle, virus, molecule and an aggregate or complex thereof. Preferably, the binding partner is an antibody or a nucleotide sequence.

Coated magnetizable microparticles with active functional groups, which are produced according to the above processes, are also provided herein. In one specific embodiment, the coated magnetizable microparticles have a mean diameter from about 10 nanometers to about 2 micrometers. In another specific embodiment, the coated magnetizable microparticles comprise a nuclei of ferrite oxide, cobalt oxide or nickel oxide. In still another specific embodiment, the coated magnetizable microparticles further comprises a binding partner that is capable binding, or capable of specifically binding, to a moiety. Exemplary binding partners include a cell, cellular organelle, virus, molecule and an aggregate or complex thereof. Preferably, the binding partner is an antibody or a nucleotide sequence.

### C. Methods for isolating and manipulating moieties

In another aspect, the present invention is directed to a method for isolating a moiety, which method comprises: a) providing coated magnetizable microparticles according to the processes described in the above Section B comprising a binding partner that is capable of binding to a moiety to be isolated; b) contacting a sample containing or suspected of containing said moiety with said coated magnetizable microparticles provided in step a) under conditions allowing binding between said moiety and said binding partner; and c) recovering said coated magnetizable microparticles from said sample with a magnetic force.

Any moiety can be isolated by the present method. For example, the moiety to be isolated can be a cell, a cellular organelle, a virus, a molecule and an aggregate or complex there of.

The coated magnetizable microparticles, with or without the bound moieties, can be recovered from the sample by any suitable methods, *e*.*g*., by a magnetic field/force using, for example, a permanent magnet or an electromagnetic chip centrifugation, or filtration.

Although the present method can be used to isolate a single moiety, it is preferably to be used in high throughput analysis and preferably a plurality of different types of moieties are isolated by using a plurality types of coated magnetizable microparticles, each type of the coated magnetizable microparticles is capable of binding to a member of the plurality types of the moieties or each type of the coated magnetizable microparticles contains a binding partner that is capable of binding to a member of the plurality types of the moieties.

A moiety in any suitable sample can be isolated. Preferably, the moiety to be isolated is contained in a fluid sample.

The present method can further comprise recovering the moiety from the coated magnetizable microparticles, *e*.*g*., by optical, chemical or other cleavage methods.

The isolation can be conducted in any suitable apparatus or device. For example, the isolation can be conducted in a liquid container such as a beaker, a flask, a cylinder, a test tube, a microcentrifuge tube, a centrifugation tube, a culture dish, a multiwell plate and a filter device or membrane. Alternatively, the isolation can be conducted in a chip format.

In still another aspect, the present invention is directed to a method for manipulating a moiety, which method comprises: a) providing coated magnetizable microparticles according to the processes described in the above Section B comprising a binding partner that is capable of binding to a moiety to be manipulated; b) coupling said moiety to said coated magnetizable microparticles provided in step a) via binding between said moiety and said binding partner to form a moiety-coated-magnetizable-microparticles complex; and c) manipulating said moiety-coated-magnetizable-microparticles complex with a magnetic force, thereby said moiety is manipulated.

When conducted in a chip format, the manipulation is effected through a combination of a structure that is external to the chip and a structure that is built-in in the chip. For example, chips and structures internal and external to the chips that are disclosed in the co-pending U.S. Patent No. 7,081,192 filed August 10,2000 and WO-A-02 28523 can be used in the present method. For example, the methods can be used on silicon, silicon dioxide, silicon nitride, plastic, glass, ceramic, photoresist or rubber chips. In addition, the methods can be used on a chemchip, *i*.*e*., on which chemical reactions are carried out, a biochip, *i*.*e*., on which biological reactions are carried out, or a combination of a biochemchip.

The physical forces used in the present methods are effected through a combination of the structure that is external to the chip and the structure that is built-in in the chip. The external structures are energy sources that can be connected to the built-in structures for energizing the built-in structures to generate a physical force such as dielectrophoresis force, magnetic force, acoustic force, electrostatic force, mechanical force or optical radiation force. The built-in structures comprise a single unit or a plurality of units. Each unit is, when energized and in combination with the external structure, capable of effecting the physical force on the moiety-bead complex. In the case of a plurality of units, the built-in structure may further comprise the means for selectively energizing any one of the plurality of units.

In one example, when magnetic force is used to manipulate a complex of a moiety (*e*.*g*., DNA molecules) and a magnetizable microparticle comprising its binding partner, the electromagnetic chip disclosed in the co-pending U.S. Patent No. 6 355, 491 filed September 17, 1999 can be used in the methods. Typically, such electromagnetic chips with individually addressable micro-electromagnetic units comprise: a substrate; a plurality of micro-electromagnetic units on the substrate, each unit capable of inducing a magnetic field upon application electric current; a means for selectively energizing any one of a plurality of units to induce a magnetic field therein. Preferably, the electromagnetic chips further comprise a functional layer coated on the surface of the chips for immobilizing certain types of molecules. In this example of magnetic manipulation of moiety-binding partner-magnetizable microparticle complexes, microelectromagnetic units are the built-in structures internal to the chip and the electrical current source that is connected to the microelectromagnetic units is the structures external to the chip. When the electric current from the external current source is applied to the microelectromagnetic units, magnetic fields will be generated in the regions around the microelectromagnetic units and magnetic forces will be produced on magnetic particles that are present in the region around the microelectromagnetic units. Typically, for the case of the manipulation force being magnetic force, the built-in structures are electromagnetic units that are incorporated on the chip and the external structures are the electrical signal sources (*e*.*g*., current sources). When the appropriately designed and fabricated electromagnetic units are energized by the electrical signal sources, magnetic fields are generated in the regions around the chip. When the coated magnetizable microparticles-binding partner-moiety complexes are subjected to such magnetic fields, magnetic forces are produced on them, and such forces are dependent on the magnetic field distribution, the magnetic properties of the coated magnetizable microparticles or the binding partner or coated magnetizable microparticles-binding partner-moiety complexes and the magnetic properties of the medium that surrounds the coated magnetizable microparticles or coated magnetizable microparticles-binding partner-moiety complexes.

Any moiety including the moieties disclosed in the above Section B can be manipulated by the present method. For example, the moiety to be manipulated can be a cell, a cellular organelle, a virus, a molecule and an aggregate or complex thereof.

The present method can be used for any type of suitable manipulation. Exemplary manipulations include transportation, focusing, enrichment, concentration, aggregation, trapping, repulsion, levitation, separation, fractionation, isolation and linear or other directed motion of the moiety.

In a preferred embodiment, the moiety is not directly manipulable by a magnetic force. In another preferred embodiment, neither the moiety nor the binding partner is directly manipulable by a magnetic force.

Although the present method can be used to manipulate a single moiety, it is preferably to be used in high throughput analysis and preferably a plurality of different types of moieties are manipulated by using a plurality types of coated magnetizable microparticles, each type of the coated magnetizable microparticles is capable of binding to a member of the plurality types of the moieties or each type of the coated magnetizable microparticles contains a binding partner that is capable of binding to a member of the plurality types of the moieties.

The present method can further comprise a step of recovering said manipulated moiety from said coated magnetizable microparticles and/or said chip, *e*.*g*., by optical, chemical or other cleavage methods.

A kit for isolating or manipulating a moiety is disclosed which comprises: a) coated magnetizable microparticles according to the processes described in the above Section B comprising a binding partner that is capable of binding to a moiety to be isolated or manipulated; and b) instruction(s) for using said coated magnetizable microparticles to isolate or manipulate said moiety.

The present methods can be used for analyzing, isolating, manipulating or detecting any types of moieties when the moieties are involved in certain processes, such as physical, chemical, biological, biophysical or biochemical processes, etc., in a chip format or non-chip format. Moieties can be cells, cellular organelles, viruses, molecules or an aggregate or complex thereof. Moieties can be pure substances or can exist in a mixture of substances wherein the target moiety is only one of the substances in the mixture. For example, cancer cells in the blood from leukemia patients, cancer cells in the solid tissues from patients with solid tumors and fetal cells in maternal blood from pregnant women can be the moieties to be isolated, manipulated or detected. Similarly, various blood cells such as red and white blood cells in the blood can be the moieties to be isolated, manipulated or detected. DNA molecules, mRNA molecules, certain types of protein molecules, or all protein molecules from a cell lysate can be moieties to be isolated, manipulated or detected.

Non-limiting examples of cells include animal cells, plant cells, fungi, bacteria, recombinant cells or cultured cells. Animal, plant cells, fungus, bacterium cells to be isolated, manipulated or detected can be derived from any genus or subgenus of the Animalia, Plantae, fungus or bacterium kingdom. Cells derived from any genus or subgenus of ciliates, cellular slime molds, flagellates and microsporidia can also be isolated, manipulated or detected. Cells derived from birds such as chickens, vertebrates such as fish and mammals such as mice, rats, rabbits, cats, dogs, pigs, cows, ox, sheep, goats, horses, monkeys and other non-human primates, and humans can be isolated, manipulated or detected by the present methods.

For animal cells, cells derived from a particular tissue or organ can be isolated, manipulated or detected. For example, connective, epithelium, muscle or nerve tissue cells can be isolated, manipulated or detected. Similarly, cells derived from an accessory organ of the eye, annulospiral organ, auditory organ, Chievitz organ, circumventricular organ, Corti organ, critical organ, enamel organ, end organ, external female genital organ, external male genital organ, floating organ, flower-spray organ of Ruffini, genital organ, Golgi tendon organ, gustatory organ, organ of hearing, internal female genital organ, internal male genital organ, intromittent organ, Jacobson organ, neurohemal organ, neurotendinous organ, olfactory organ, otolithic organ, ptotic organ, organ of Rosenmüller, sense organ, organ of smell, spiral organ, subcommissural organ, subfornical organ, supernumerary organ, tactile organ, target organ, organ of taste, organ of touch, urinary organ, vascular organ of lamina terminalis, vestibular organ, vestibulocochlear organ, vestigial organ, organ of vision, visual organ, vomeronasal organ, wandering organ, Weber organ and organ of Zuckerkandl can be isolated, manipulated or detected. Preferably, cells derived from an internal animal organ such as brain, lung, liver, spleen, bone marrow, thymus, heart, lymph, blood, bone, cartilage, pancreas, kidney, gall bladder, stomach, intestine, testis, ovary, uterus, rectum, nervous system, gland, internal blood vessels, etc. can be isolated, manipulated or detected. Further, cells derived from any plants, fungi such as yeasts, bacteria such as eubacteria or archaebacteria can be isolated, manipulated or detected. Recombinant cells derived from any eucaryotic or prokaryotic sources such as animal, plant, fungus or bacterium cells can also be isolated, manipulated or detected. Cells from various types of body fluid such as blood, urine, saliva, bone marrow, sperm or other ascitic fluids, and subfractions thereof, *e*.*g*., serum or plasma, can also be isolated, manipulated or detected.

Isolatable, manipulatable or detectable cellular organelles include nucleus, mitochondria, chloroplasts, ribosomes, ERs, Golgi apparatuses, lysosomes, proteasomes, secretory vesicles, vacuoles or microsomes. Isolable, manipulable or detectable viruses include intact viruses or any viral structures, *e*.*g*., viral particles, in the virus life cycle that can be derived from viruses such as Class I viruses, Class II viruses, Class III viruses, Class IV viruses, Class V viruses or Class VI viruses.

Isolable, manipulable or detectable molecules can be inorganic molecules such as ions, organic molecules or a complex thereof. Non-limiting examples of ions include sodium, potassium, magnesium, calcium, chlorine, iron, copper, zinc, manganese, cobalt, iodine, molybdenum, vanadium, nickel, chromium, fluorine, silicon, tin, boron or arsenic ions. Non-limiting examples of organic molecules include amino acids, peptides, proteins, nucleosides, nucleotides, oligonucleotides, nucleic acids, vitamins, monosaccharides, oligosaccharides, carbohydrates, lipids or a complex thereof.

Any amino acids can be isolated, manipulated or detected by the present methods. For example, a D- and a L-amino-acid can be isolated, manipulated or detected. In addition, any building blocks of naturally occurring peptides and proteins including Ala (A), Arg (R), Asn (N), Asp (D), Cys (C), Gln (Q), Glu (E), Gly (G), His (H), Ile (I), Leu (L), Lys (K), Met (M), Phe (F), Pro (P) Ser (S), Thr (T), Trp (W), Tyr (Y) and Val (V) can be isolated, manipulated or detected.

Any proteins or peptides can be isolated, manipulated or detected by the present methods. For example, membrane proteins such as receptor proteins on cell membranes, enzymes, transport proteins such as ion channels and pumps, nutrient or storage proteins, contractile or motile proteins such as actins and myosins, structural proteins, defense protein or regulatory proteins such as antibodies, hormones and growth factors can be isolated, manipulated or detected. Proteineous or peptidic antigens can also be isolated, manipulated or detected.

Any nucleic acids, including single-, double and triple-stranded nucleic acids, can be isolated, manipulated or detected by the present methods. Examples of such nucleic acids include DNA, such as A-, B- or Z-form DNA, and RNA such as mRNA, tRNA and rRNA.

Any nucleosides can be isolated, manipulated or detected by the present methods. Examples of such nucleosides include adenosine, guanosine, cytidine, thymidine and uridine. Any nucleotides can be isolated, manipulated or detected by the present methods. Examples of such nucleotides include AMP, GMP, CMP, UMP, ADP, GDP, CDP, UDP, ATP, GTP, CTP, UTP, dAMP, dGMP, dCMP, dTMP, dADP, dGDP, dCDP, dTDP, dATP, dGTP, dCTP and dTTP.

Any vitamins can be isolated, manipulated or detected by the present methods. For example, water-soluble vitamins such as thiamine, riboflavin, nicotinic acid, pantothenic acid, pyridoxine, biotin, folate, vitamin B₁₂ and ascorbic acid can be isolated, manipulated or detected. Similarly, fat-soluble vitamins such as vitamin A, vitamin D, vitamin E, and vitamin K can be isolated, manipulated or detected.

Any monosaccharides, whether D- or L-monosaccharides and whether aldoses or ketoses, can be isolated, manipulated or detected by the present methods. Examples of monosaccharides include triose such as glyceraldehyde, tetroses such as erythrose and threose, pentoses such as ribose, arabinose, xylose, lyxose and ribulose, hexoses such as allose, altrose, glucose, mannose, gulose, idose, galactose, talose and fructose and heptose such as sedoheptulose.

Any lipids can be isolated, manipulated or detected by the present methods. Examples of lipids include triacylglycerols such as tristearin, tripalmitin and triolein, waxes, phosphoglycerides such as phosphatidylethanolamine, phosphatidylcholine, phosphatidylserine, phosphatidylinositol and cardiolipin, sphingolipids such as sphingomyelin, cerebrosides and gangliosides, sterols such as cholesterol and stigmasterol and sterol fatty acid esters. The fatty acids can be saturated fatty acids such as lauric acid, myristic acid, palmitic acid, stearic acid, arachidic acid and lignoceric acid, or can be unsaturated fatty acids such as palmitoleic acid, oleic acid, linoleic acid, linolenic acid and arachidonic acid.

### D. Preferred embodiment

In one specific embodiment, the present invention is directed to a suspension-polymerization method used for surface-coating and functionalizing magnetizable microparticles (or magnetic micro-beads), which method comprises:
(1) under vigorous ultrasonication and stirring, dispersing mono-dispersed magnetic micro-beads with the certain diameter between 5 and 1000 nanometers in organic solvent containing a surfactant;
(2) adding coating monomers, functionalization reagent, initiator, coupling agent as defined above and cross-linking agent into the organic solvent containing magnetic micro-beads;
(3) stirring the mixture vigorously for 30min to make the monomer disperse evenly and distribute on the micro-beads' surface under purging with a stream of nitrogen; then lowering stirring velocity to 30rpm and raising the reaction temperature to 80°C and maintaining nitrogen atmosphere for 12 hours;
(4) after the completion of the polymerization, stirring the reaction mixture on the magnetic stirrer until the deposition of the magnetic resin and discarding the clear supernatant; and
(5) filtering the magnetic resin washed with deionized water followed by the acetone to obtain the coated magnetic micro-beads modified with functional groups.

In this embodiment, the quantity of the reagents can be changed to meet the demands. All the organic monomers are in an amount from 1:400 to 1:1 by weight with respect to the amount of the magnetic micro-beads. The amount of all the monomers can be as follows: coating monomer 0∼80%, coupling agent 1∼10%, cross-linking agent 10∼80%, functionalization reagent 5∼40% and initiator 1∼5%.

In this embodiment, suitable coating monomers includes but are not limited to: acrylic acid, methacrylic acid, methyl methacrylate, 2-hydroxyethyl methacrylate, glycoldimethylacrylate, ethylene glycol diacrylate, ethylene glycol dimethacrylate, diethylene glycol methacrylate, diethylene glycol acrylate, diethylene glycol dimethacrylate, diethylene glycol diacrylate, diethylene glycol methacrylate, diethylene glycol acrylate, triethylene glycol dimethacrylate, trimethylolpropane trimethacrylate, pentaerythritol triacrylate, styrene, divinylbenzene and a mixture thereof These monomers can be used independently or together. And it is preferable to be used with a cross-linking agent.

In this embodiment, suitable cross-linking agents include but are not limited to: diethylene glycol dimethacrylate, diethylene glycol diacrylate, ethylene glycol diacrylate, ethylene glycol dimethacrylate, trimethylolpropane trimethacrylate, pentaerythritol triacrylate and divinylbenzene. Excellent coating result can be achieved when they react with suited coating monomers. These cross-linking agents can be used independently or together.

In this embodiment, suitable organic solvent can be toluene, dimethylbenzene, tetrahydrofuran, or ethanol used alone or mixed with water. These organic solvents can be used independently or together.

In this embodiment, an anionic surfactant, such as dodecyl sulfonic acid sodium salt or sodium dodecyl benzene sulfonate, or a nonionic surfactant, such as alkylphenolpolyoxyethene ether, can be used to disperse the magnetic micro-beads in the organic solvent. Before the polymerization, the reaction system can be stirred vigorously and dispersed with ultrasonication to prevent the nano-particles from aggregating. After the polymerization begins, the stirring velocity can be lowered to prevent the particles colliding with each other. These surfactants can be used independently or together.

In this embodiment, suitable initiator can be benzoyl peroxide or 2,2'-azobisisobutyronitrile. These initiators can be used independently or together.

The coated magnetic micro-beads' surface should be modified with functional groups to couple with desired moieties, *e*.*g*., biological molecules, more effectively. Carboxyl, amino, hydroxyl, sulfhydryl, hydrosulfuryl, epoxy, ester, alkene, alkyne, alkyl, aromatic, aldehyde, ketone, sulfate, amide, urethane group(s), or their derivatives thereof can be used as functional groups. The magnetic micro-beads can be bound with biological molecules covalently through these functional groups. This link is more stable and not easily broken off. In one embodiment, the coating monomers can contain functional groups and their double bonds are used as functional reagents. During the coating process, the monomers containing the functional groups are added into the system. The coating and functionalization process are accomplished simultaneously. In this embodiment, suitable functionalization reagents includes but are not limited to: acrylic acid, methacrylic acid, glycidyl acrylate, pentaerythritol diacrylate and methacrolein.

The difference of polarity between the magnetic nano-particles and coating polymer should be large. Therefore, it is sometimes important to add coupling reagents to let the polymer coat on the micro-beads' surface completely. The coupling reagent is selected from the group consisting of bis(2-hydroxyethyl methacrylate) phosphate, bis(trimethylolpropane diacrylate) phosphate, and bis(pentaerythritol triacrylate) phosphate. These coupling reagents can be used independently or together. One end of these coupling agents can be a phosphoryl group with strong polarity, which can attach strongly to ferrous oxide, and the other end can be a double bond, which can attach to and copolymerize with monomers. The monomers can be adsorbed on the surface of the nano-beads polymerize. Thus, one even polymer layer can be obtained on the surface with the formed network, while the monomers do not form new polymer particles among themselves. By adding functionalization reagent with a double bond, the process of coating and functionalization can be carried out in one step.

In another specific embodiment, the present invention is directed to the coated magnetic magnetizable microparticles (or magnetic micro-beads) prepared according to the above process. The coated magnetic micro-beads or nano-beads containing functional groups are polymers having composite materials, whose inner nucleus is comprised of metal oxide and whose outer layer is comprised of polymer or biological macromolecules, which contains certain functional groups. The magnetic response should not be remarkably reduced by the coating and modification process. The σₛ of the prepared magnetic nanocrystals is high, and the remanence and coercivity are low. The magnetic micro-beads can be spherically shaped and can have narrow diameter distribution. The diameter of the coated magnetic micro-beads can be uniform, and the mean diameter of them can be from 10 nanometers to 2 micrometers. They can be evenly dispersed in aqueous solution without precipitation and flocculation and can withstand erosion in a biological environment.

In the coating process, the monomers polymerize with the magnetic micro-beads as nuclei. The shell layer thickness and mean density of the coated nano-beads can be controlled by changing the quantity of the coating monomers, functionalization reagents, coupling agents and cross-linking agents. The coated magnetic micro-beads with the mean diameter from 10 nanometers to 2 micrometers can be prepared by changing the amount of the monomers using the magnetic nano-crystals with the mean diameter from 5 to 1000 nanometers as nuclei.

In this embodiment, it is preferable to use the Fe₃O₄ or γ-Fe₂O₃ as nuclei in the coating process. Their densities are larger than 4kg/m³. To keep the coated magnetic micro-beads suspended in the solution and prevent them from being eroded or dissolved, they can be coated with organic polymer to reduce their mean density to keep them suspended in the solution for a long time.

This embodiment also relates to the application of the magnetic micro-beads in the purification, concentration, separation and test and their application as the carriers for the directed manipulation of the various moieties, *e*.*g*., biological molecules in various formats, *e*.*g*., on micro-electromagnetic unit array chips.

The magnetic micro-beads prepared in this embodiment can be used in separation of biological materials, as carriers for the directed manipulation and magnetic probe to mark the biological molecules, such as DNA, RNA, polypeptide, protein (enzyme, antigen, antibody) and cells. This magnetic micro-bead probe can be used widely for marking the biological molecules, purification, concentration and separation in the biological research, such as the immunity diagnosis, construction of the cDNA library and the carriers for the directed manipulation of the biological molecules in micro-electromagnetic unit array chips.

The binding between the functional groups of the present coated magnetic micro-beads and biological molecules can direct or indirect binding. The magnetic micro-beads are preferably be sterilized before use. They can be stored at 4°C (pH 4-10) for a long time.

In one exemplary direct binding method, the magnetic micro-beads are dispersed in a phosphate solution. The biological molecules are added. The system should be kept at alkaline condition if the functional groups are epoxy groups. If the functional groups are carboxyl groups, 1-ethyl-3-(3-dimethylaminopropyl) carbodiimide can be added. When the amino groups are bound with biological molecules, they can be converted to aldehyde groups through adding glutaraldehyde before their binding with biological molecules. After the reaction under the slow stirring for several hours, the magnetic micro-beads are immobilized and the clear supernatant is discarded. The immobilized micro-beads are washed with phosphate buffer three times to obtain the biological molecules bound with the magnetic micro-beads. These marked biological molecules can be bound with specific biological molecules differentially so as to separate, extract and test these biological molecules. For example, if an antibody is immobilized on the surface of the magnetic micro-beads, the bio-conjugate can be bound with the specific antigen to separate this specific antigen.

In one exemplary indirect binding method, a specific linking system can be used to separate biological molecules, such as biotin and avidin. In this example, the functional groups on the surface of the magnetic micro-beads are bound with avidin or streptavidin. At the same time, the target biological molecules are bound with biotin. Then the target molecules can be separated efficiently and quickly from the bulk solution through the specific binding between biotin and avidin. The bioconjugate can be separated through the magnetic force. Then the target molecules can be washed. This process can be used in clinical diagnosis, and analysis on DNA-chip and protein chips.

The mono-dispersed magnetic nano-crystals with certain diameter between 5 and 1000 nanometers can be coated with polymer using the suspension-polymerization method and modified with functional groups by adding the functional monomer during the polymerization. The density and properties of hydrophile and hydrophobia can be improved and the nanoparticles can be prevented from being corrupted. In the coating process, the magnetism is not changed remarkably, no new nuclei are formed and the magnetic micro-beads do not aggregate. The coated magnetic micro-beads with mean diameter from 10 nanometers to 2 micrometers can be prepared by changing the amount of the monomers. The magnetic micro-beads have narrow diameter distribution and excellent monodispersity and keep suspending in aqueous solution. For example, biological molecules, such as antigen, antibody, DNA, FNA, protein, enzyme and cells, can be immobilized on the surface of the magnetic micro-beads through specific binding. The coupled biological molecules can maintain their biological activities. The magnetic micro-beads can be used in the purification, concentration, separation and test of biological materials and be used as carriers for the directed manipulation of the biological molecules in micro-electromagnetic unit array chips.

### E. Examples

### Example 1. Surface coating with 2-hydroxyethyl methylmethacrylate and epoxy functionalization of 10 nm magnetic Fe₂O₄ nano-crystals

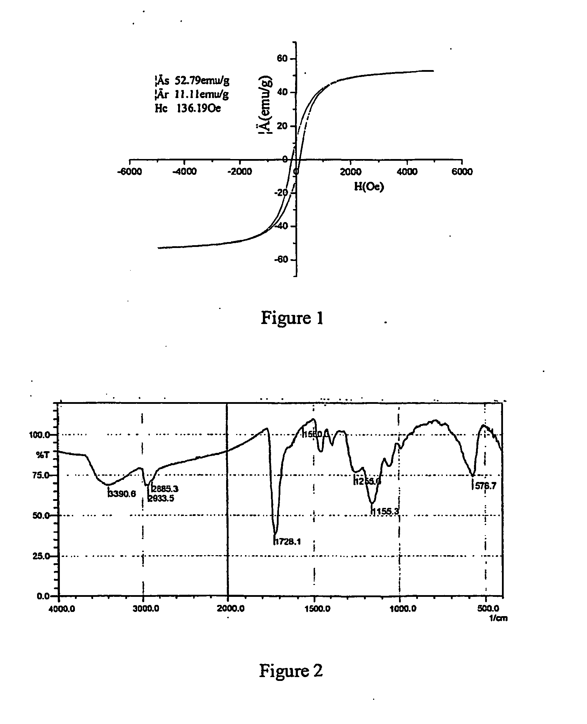

To a three-necked flask equipped with a stirrer, condenser, and thermostat containing 500ml of toluene and 0.816g sodium lauryl benzene sulfonate, 3.274g superparamagnetic nanocrystals were added. The nanocrystals were well dispersed into toluene by ultrasound for 0.5h and violent agitation. A mixture of 0.242g initiator benzoylperoxide (BPO), 2.5ml monomer 2-hydroxyethyl methacrylate (Acros), 1.5 ml cross-linking trimethylolpropanetriacrylate (Acros), 0.6ml coupling agent bis-(2-hydroxyethyl methacrylate) phosphate and 1.2ml functionalization agent methacrylic acid (Acros) was added into the flask. The mixture was stirred violently for 30min under purging with a stream of nitrogen. Then the stirring velocity was lowered to 30rpm, and the reaction temperature was raised to 80°C and maintained for 12h under nitrogen atmosphere. After the completion of the polymerization, the coated superparamagnetic nanocrystals were separated by magnetic field and then washed successively with toluene, acetone, ethanol and deionized water to remove the residual surfactant and unreacted reagents. Finally the coated superparamagnetic nano-beads were dispersed into the Tris-EDTA buffer (pH 6.5) and stored at 4°C. The prepared magnetic micro-beads have excellent hydrophile property and can suspend in water or aqueous solution. They contain epoxy groups on the surface and can bind with biological molecules containing amino groups. The FT-IR spectra of the coated Fe₃O₄ magnetic nanoparticles are shown in Figure 2, which shows the spectrum of coated nanocrystals. It can be seen that there is a series of absorption bands in Figure 2. Besides the absorption peak of hydroxyl group from 2-hydroxyethyl methacrylate or acrylic acid at 3390 cm⁻¹ and the absorption peak of Fe₃O₄ at 576 cm⁻¹, other observed absorption peaks include: -CH₂- peak at 2933 cm⁻¹; -C=C- peak at 1560 cm⁻¹; -CH- peak at 2885 cm⁻¹; epoxy group peak at 1255 cm⁻¹; -C=O group at 1728 cm⁻¹; and -P=O group at 1057 cm⁻¹. The existence of these organic groups proved successful coating and functionalization.

### Example 2. Surface coating with polystyrene and carboxyl functionalization on 50 nm magnetic Y -Fe₂O₃ nano-crystals

The polymerization-blocking agents contained in the styrene monomer must be removed before the polymerization. 5 ml styrene were washed with 4mol/L NaOH solution to remove the polymerization-blocking agents, followed by deionized water wash to keep the monomer neutral. The coating process is similar to the process used in example 1.

To a three-necked flask equipped with a stirrer, condenser, and thermostat containing 500ml of toluene and 0.813g dodecyl sulfonic acid sodium salt were added 2.974g superparamagnetic γ-Fe₂O₃ nanocrystals with the diameter of 50nm. The nanocrystals were well dispersed into toluene by ultrasound for 0.5h and violent agitation. A mixture of 0.208g initiator benzoylperoxide (BPO), 5ml monomer styrene, 3ml cross-linking divinylbenzene, 0.5ml coupling agent Bis(trimethylolpropane diacrylate) phosphate and 1.5ml functionalized agent methacrylic acid was added into the flask. The mixture was stirred violently for 30min under purging with a stream of nitrogen. Then the stirring velocity was lowered to 30rpm, and the reaction temperature was raised to 80°C and maintained for 12h under nitrogen atmosphere. After the completion of the polymerization, the coated superparamagnetic nanocrystals were separated by magnetic field and then washed successively with toluene, acetone, ethanol and deionized water to remove the residual surfactant and unreacted reagents. Finally the coated superparamagnetic nano-beads were dispersed into the 10ml Tris-EDTA buffer (pH 6.5) and stored at 4°C. The prepared magnetic micro-beads containing carboxyl groups have excellent hydrophile property and can suspend in water.

### Example 3. Surface coating with methyl methacrylate and aldehyde functionalization on 200 nm magnetic Fe₃O₄ nano-crystals

To a three-necked flask equipped with a stirrer, condenser, and thermostat containing 500ml of xylene and 0.614g alkylphenolpolyoxyethylene ether were added 5.584g superparamagnetic Fe₃O₄ nanocrystals with the diameter of 200nm. The nanocrystals were well dispersed into dimethylbenzene by ultrasound for 0.5h and violent agitation. A mixture of 0.235g 2,2'-azobisisobutyronitrile, 2.5ml monomer methyl methacrylate, 2ml cross-linking pentaerythritol dimethacrylate, 0.4ml coupling agent bis-(2-hydroxyethyl methacrylate) phosphate and 1.5ml functionalized agent methacrolein was added into the flask. The mixture was stirred violently for 30min under purging with a stream of nitrogen. Then the stirring velocity was lowered to 30rpm, and the reaction temperature was raised to 80°C and maintained for 12h under nitrogen atmosphere. After the completion of the polymerization, the coated superparamagnetic nanocrystals were separated by magnetic field and then washed successively with toluene, acetone, ethanol and deionized water to remove the residual surfactant and unreacted reagents. Finally the coated superparamagnetic nano-beads were dispersed into the 10ml Tris-EDTA buffer (pH 6.5) and stored at 4°C. The prepared magnetic micro-beads contain aldehyde groups.

### Example 4. Surface coating with 2-hydroxyethyl methacrylate and hydroxyl functionalization on 100 nm magnetic Y -Fe₂O₃ nano-crystals

To a three-necked flask equipped with a stirrer, condenser, and thermostat containing 500ml of 80% ethanol and 20% water, and 0.593g alkylphenolpolyoxyethylene ether were added 2.934g superparamagnetic Y -Fe₂O₃ nanocrystals with the diameter of 200nm. The nanocrystals were well dispersed into ethanol solution by ultrasonication for 0.5h and violent agitation. A mixture of 0.247g initiator benzoylperoxide (BPO), 2ml monomer 2-hydroxyethyl methacrylate, 3.5ml cross-linking pentaerythritol triacrylate, 0.5ml coupling agent bis(pentaerythritol triacrylate) phosphate was added into the flask. The mixture was stirred violently for 30min under purging with a stream of nitrogen. Then the stirring velocity was lowered to 30rpm, and the reaction temperature was raised to 76°C and maintained for 12h under nitrogen atmosphere. After the completion of the polymerization, the coated superparamagnetic nano-crystals were separated by magnetic field and then washed successively with toluene, acetone, ethanol and deionized water to remove the residual surfactant and unreacted reagents. Finally the coated superparamagnetic nano-beads were dispersed into the 10ml Tris-EDTA buffer (pH 6.5) and stored at 4°C. The prepared magnetic micro-beads contain hydroxyl groups.

### Example 5. Surface coating with pentaerythritol trimethacrylate and epoxy functionalization on 1000 nm magnetic Fe₃O₄ nano-crystals

To a three-necked flask equipped with a stirrer, condenser, and thermostat containing 500ml of toluene and 0.583g sodium dodecyl benzene sulfonate were added 2.836g superparamagnetic Fe₃O₄ nanocrystals with the diameter of 1000nm. The nanocrystals were well dispersed into toluene by ultrasound for 0.5h and violent agitation. A mixture of 0.227g 2,2'-azobisisobutyronitrile, 2.2ml monomer pentaerythritol trimethacrylate, 1.5ml cross-linking trimethylolpropanetriacrylate, 0.4ml coupling agent bis-(2-hydroxyethyl methacrylate) phosphate and 1.8ml functionalized agent glycidyl acrylate was added into the flask. The mixture was stirred violently for 30min under purging with a stream of nitrogen. Then the stirring velocity was lowered to 30rpm, and the reaction temperature was raised to 76°C and maintained for 12h under nitrogen atmosphere. After the completion of the polymerization, the coated superparamagnetic nanocrystals were separated by magnetic field and then washed successively with toluene, acetone, ethanol and deionized water to remove the residual surfactant and unreacted reagents. Finally the coated superparamagnetic nano-beads were dispersed into the 10ml Tris-EDTA buffer (pH 6.5) and stored at 4°C. The prepared magnetic micro-beads contain epoxy groups.

### Example 6. Application for the directed manipulation of biological molecules in micro-electromagnetic unit array chips

The magnetic nano-beads obtained from example 3 were bound with a target DNA. In micro-electromagnetic unit array chips (*See e*.*g*., U.S. Patent No. 6,355,491), the target DNA samples were concentrated in the chosen cell to realize the directed manipulation of the DNA molecules. The original micro-electromagnetic unit array chips have 16 individually addressable electromagnetic poles. The electromagnetic field can be controlled via changing the currency of individual electromagnetic pole. There are 16 individually addressable electromagnetic poles under the reactor made with UV curving glue on the micro-electromagnetic unit array chips. When the electromagnetic pole was electrified, the magnetic micro-beads suspended in the solution were magnetized by the electromagnetic field to concentrate at the chosen electromagnetic pole. The directed manipulation can be realized for the magnetic micro-beads and the biological molecules bound with micro-beads. This had been clearly observed under the microscope as shown in Figure 3.

### Example 7. Application for immunization analysis

Conventional sandwich assay was used in this study. To prevent protein loss due to adsorption to the eppendorf tube, the 1.5 ml eppendorf tube was coated with 1% bovine serum albumin (BSA) and kept at 4°C for overnight under obturation. 50 µl of the suspension of the magnetic nano-beatis (20µg/µl) prepared in Example 5 were added into the tube, followed by 200µl of goat anti-human IgG sodium carbonate buffer. The reaction lasted for 2 hours under vibration at 37°C. Then the magnetic nano-beads were immobilized by magnetic stand and the clear supernatant was discarded. The immobilized nano-beads were washed with phosphate buffer saline ( PBS, Na₂HPO₄8.1mM, NaH₂PO₄1.9mM, NaCl 0.14M, Tween 20 0.05%, pH7.4) for three times, 5 min each. The nano-beads were blocked with 1% BSA solution and the reaction lasted for 1 hour at 37°C. This is necessary for the removal of the non-specifically adsorbed antigen. Then 200µl antigen, *i*.*e*., human immunoglobulin IgG, were added into the closed eppendorftube and the reaction lasted for 1 hour at 37°C. The nano-beads were washed with phosphate buffer for three times, 5 min each. Two hundred µl rabbit anti-human IgG marked with FITC were added into the eppendorf tube and the reaction lasted for 2 hours at 37°C. Then the magnetic micro-beads were washed with PBS two times, followed by a wash with deionized water. The magnetic nano-beads were diluted with 50µl PBS solution.

The readout of immunoassay with labeled antibody on the glass slide and the calculation of fluorescent intensity were performed on a Scanner Array 4000 equipped with two-laser powers. Glass microscope chips were first soaked in chrome pickle for 2h, rinsed with copious deionized water and dried in a stream of nitrogen. They were successively cleaned by ultrasonic waves for 15min each in hexane, acetone and ethanol, dried at 80°C for 5min. Then the obtained suspension containing magnetic micro-beads was dispersed dropwise onto the activated surface of the chip using a robotic microdispersing system. The volume of each distribution is in picoliter order of magnitude. The diameter of each spot was about 300µm and the distance between two spots was 800µm. Then the chips were dried in a stream of nitrogen. Then the fluorescent imaging of the chips was implemented on the Scanner Array 4000. The 532nm laser line was used as the excitation power.

### Example 8. Adsorption of genomic DNA using coated magnetic nano-beads

The coated magnetic nano-beads prepared in the above examples can be used to extract, separate and purify DNA from biological samples and the yield of DNA can be controlled by changing the use of salt or the organic solvent and its concentration and pH. The steps in this process are as follows. To a sterilized eppendorf tube containing 40µl of the magnetic nano-beads suspension (15mg/ml) were added 15µl of 0.4µg/µl λDNA (Hind□ digest) marker. The mixture was resuspended by vortexing gently for 15s. Eighty µl of 4mol/L NaI solution were added into the tube. The mixture was vortexed gently for 30s and incubated for 3min. Then 100µl of isopropanol was added into the tube. The mixture was vortexed gently for 5s and incubated for 2min. The nano-beads were immobilized on a magnetic stand and the supernatant was discarded. The magnetic nano-beads adsorbing genomic DNA were washed with isopropanol twice. Then 50µl Tris-EDTA (pH 8.0, Tris HCl 10mmol/L, EDTA 1mmol/L) were added into the tube and incubated at 62°C for 12min to elute genomic DNA. The magnetic nano-beads were separated from the TE solution. The separated TE solution containing genomic DNA was analyzed with agarose gel electrophoresis. It was shown in Fig. 4 that the yields of DNA with different molecular weight are clearly different.

### Example 9. Separation of genomic DNA of E.coli. using the coated magnetic nano-beads

The sample was *E.coli.* bacteria which were cultured overnight. 1 ml of the cell culture was placed in the uperized eppendorf tube and then centrifuged at 3000rpm for 30s. The clear supernatant was discarded. 500 µl of SDS degradation fluid were added. The mixture was agitated gently and placed at the room temperature for 10min. Then 50ul of the 15mM suspension of the magnetic nanoparticles were added, followed by 300ul of acetone. The mixture was resuspended by vortexing gently for 30s and incubated for 5min. The magnetic nanoparticles were immobilized by Promaga magnetic stand and the clear supernatant was discarded. The immobilized nanoparticles were washed with 70% aqueous ethanol twice. Then 100ul of TE (pH 8.0) were added and the mixture was kept at 65°C in a water bath for 10min. The magnetic nanoparticles were immobilized by magnetic stand and the eluent was collected and analyzed by agarose gel electrophoresis and ultraviolet spectroscopy.

Figure 5 shows the results of agarose gel electrophoresis of genomic DNA isolated from *E.coli* using different separation technique and differently disposed magnetic micro-beads. The OD₂₆₀/OD₂₈₀ ratios of all the samples are larger than 1.8 when the eluents were diluted. The yield of genomic DNA is 30ugl/ul. Compared with conventional methods, the magnetic nano-beads method separate genomic DNA with similar yield and purity. This method has the following advantages: (1) it is simple and only takes 20 min to complete the whole process; (2) it is suitable for automation processes; (3) this method can be completed without centrifugation procedure and the utility of poisonous agents; and (4) this process can be resumed after any interruption among 24 hours at room temperature without restarting.

## Claims

1. A process for producing coated magnetizable microparticles with active functional groups, which process comprises:
a) dispersing magnetizable microparticles with a diameter ranging from 5 to 1,000 nanometers in an organic solvent containing a surfactant;
b) adding a coupling agent, coating monomers, a functionalization reagent, a cross-linking agent and an initiator to said organic solvent containing said dispersed magnetizable microparticles, allowing attachment of said coupling agent to the surface of said magnetizable microparticles and dispersing said coating monomers evenly on the surface of said magnetizable microparticles with said attached coupling agent;
c) initiating and completing polymerization of said coating monomers to form coated magnetizable microparticles with active functional groups in the absence of oxygen, whereby polymers of said coating monomers are attached to the surface of said coated magnetizable microparticles via said coupling agent, multiple said polymers are crosslinked together via said cross-linking agent and said functionalization reagent is linked to said polymers, cross-linking agent and/or coupling agent so that at least one functional group of said functionalization reagent remains available,
wherein the coupling reagent is selected from the group consisting of bis(2-hydroxyethyl methacrylate)phosphate, bis(trimethylolpropane diacrylate)phosphate, and bis(pentaerythritol triacrylate)phosphate.

2. The process of claim 1, wherein the magnetizable microparticles comprise a transition metal composition or an alloy thereof.

3. The process of claim 2, wherein the transition metal is selected from the group consisting of iron, nickel, copper, cobalt, manganese, tantalum, zirconium, nickel-iron alloy and cobalt-tantalum-zirconium (CoTaZr) alloy.

4. The process of claim 1, wherein the magnetizable microparticles comprise a metal oxide.

5. The process of any of the preceding claims, wherein the organic solvent is selected from the group consisting of toluene, dimethylbenzene, tetrahydrofuran and ethanol.

6. The process of any of the preceding claims, wherein the surfactant is dodecyl sulfonic acid sodium salt or sodium dodecyl benzene sulfonate.

7. The process of any of the preceding claims, wherein the concentration of the surfactant in the organic solution ranges from about 0.1% (v/v) to about 5% (v/v).

8. The process of any of the preceding claims, wherein the coating monomers are selected from the group consisting of acrylic acid, methacrylic acid, methyl methacrylate, 2-hydroxyethyl methacrylate, glycoldimethylacrylate, ethylene glycol diacrylate, ethylene, glycol dimethacrylate, diethylene glycol methacrylate, diethylene glycol acrylate, diethylene glycol dimethacrylate, diethylene glycol diacrylate, triethylene glycol dimethacrylate, trimethylolpropane trimethacrylate, pentaerythritol triacrylate, styrene, divinylbenzene and a mixture thereof.

9. The process of any of the preceding claims, wherein the functionalization reagent is selected from the group consisting of acrylic acid, methacrylic acid, glycidyl acrylate, pentaerythritol diacrylate and methacrolein.

10. The process of any of the preceding claims, wherein the cross-linking agent is selected from the group consisting of diethylene glycol dimethacrylate, diethylene glycol diacrylate, ethylene glycol diacrylate, ethylene glycol dimethacrylate, trimethylolpropane trimethacrylate, pentaerythritol triacrylate and divinylbenzene.

11. The process of any of the preceding claims, wherein the initiator is benzoyl peroxide or 2,2'-azobisisobutyronitrile.

12. The process of any of the preceding claims, wherein the ratio between the sum of the coupling agent, the coating monomers, the functionalization reagent and the cross-linking agent versus the magnetizable microparticles ranges from about 1:400 (w/w) to about 1:1 (w/w).

13. The process of any of the preceding claims, wherein the percentages of coating monomers, coupling agent, cross-linking agent, functionalization reagent and initiator in the sum of the substances are: from about 0% to about 80% (v/v) coating monomers, from about 1% to about 10% (v/v) coupling agent, from about 10% to about 80% (v/v) cross-linking agent, from about 5% to about 40% (v/v) functionalization reagent and from about 1% to about 5% (w/v) initiator.

14. Coated magnetizable microparticles with active functional groups, which are obtainable according to the process of any of claims 1 to 13.

15. The coated magnetizable microparticles of claim 14, which have a mean diameter from 10 nanometers to 2 micrometers.

16. The coated magnetizable microparticles of claim 14 or 15, which comprise a nuclei selected from the group consisting of ferrite oxide, cobalt oxide and nickel oxide.

17. The coated magnetizable microparticles of any of claims 14 to 16, which further comprise a binding partner that is capable of binding to a moiety.

18. The coated magnetizable microparticles of claim 17, wherein the binding partner is an antibody or a nucleotide sequence.

19. The coated magnetizable microparticles of claim 17, wherein the binding partner is selected from the group consisting of a cell, cellular organelle, virus, molecule and an aggregate or complex thereof.

20. A method for isolating a moiety, which method comprises:
a) providing coated magnetizable micorparticles of any of claims 14 to 19 comprising a binding partner that is capable of binding to a moiety to be isolated;
b) contacting a sample containing or suspected of containing said moiety with said coated magnetizable microparticles provided in step a) under conditions allowing binding between said moiety and said binding partner; and
c) recovering said coated magnetizable microparticles from said sample with a magnetic force.

21. The method of claim 20, further comprising recovering the moiety from the coated magnetizable microparticles.

22. The method of claim 20 or 21, wherein the isolation is conducted on a chip.

23. A method for manipulating a moiety, which method comprises:
a) providing coated magnetizable microparticles of any of claims 14 to 19 comprising a binding partner that is capable of binding to a moiety to be manipulated;
b) coupling said moiety to said coated magnetizable microparticles provided in step a) via binding between said moiety and said binding partner to form a moiety-coated-magnetizable-microparticles complex; and
c) manipulating said moiety-coated-magnetizable-microparticles complex with a magnetic force,
thereby said moiety is manipulated.

24. The method of claim 23, further comprising recovering the moiety from the coated magnetizable microparticles.

25. The method of claim 23 or 24, wherein the manipulation is conducted on a chip.

26. The method of any of claims 23 to 25, wherein the manipulation is selected from the group consisting of transportation, focusing, enrichment, concentration, aggregation, trapping, repulsion, levitation, separation, fractionation, isolation and linear or other directed motion of the moiety.

## Patentansprüche

1. Ein Verfahren zur Herstellung beschichteter magnetisierbarer Mikropartikel mit aktiven funktionellen Gruppen, wobei das Verfahren umfasst:
a) Dispergieren magnetisierbarer Mikropartikel mit einem Durchmesser im Bereich von 5 bis 1,000 nm in einem organischen Lösungsmittel, welches ein grenzflächenaktives Mittel enthält;
b) Zugabe eines Kupplungsmittels, von Beschichtungsmonomeren, eines Funktionalisierungsreagens, eines Vernetzungsmittels und eines Initiators zu dem organischen Lösungsmittel, welches die dispergierten magnetisierbaren Mikropartikel enthält, Zulassen einer Bindung des Kupplungsmittels an die Oberfläche der magnetisierbaren Mikropartikel und Dispergieren der Beschichtungsmonomere gleichmäßig auf der Oberfläche der magnetisierbaren Mikropartikel mit dem daran gebundenen Kupplungsmittel;
c) Initiieren und Vervollständigen der Polymerisation der Beschichtungsmonomere, um beschichtete magnetisierbare Mikropartikel mit aktiven funktionellen Gruppen in Abwesenheit von Sauerstoff zu bilden, wobei Polymere der Beschichtungsmonomere an die Oberfläche der beschichteten magnetisierbaren Mikropartikel über das Kupplungsmittel gebunden sind, eine Vielzahl der Polymere über das Vernetzungsmittel vernetzt sind und das Funktionalisierungsreagens an die Polymere, das Vernetzungsmittel und/oder das Kupplungsmittel gebunden ist, so dass mindestens eine funktionelle Gruppe des Funktionalisierungsreagens verfügbar bleibt,
wobei das Kupplungsreagens aus der Gruppe bestehend aus bis(2-Hydroxyethylmethacrylat)phosphat, bis(Trimethylolpropandiacrylat)phosphat und bis(Pentaerythritittriacrylat)phosphat ausgewählt ist.

2. Das Verfahren gemäß Anspruch 1, wobei die magnetisierbaren Mikropartikel eine Übergangsmetallzusammensetzung oder eine Legierung davon umfassen.

3. Das Verfahren gemäß Anspruch 2, wobei das Übergangsmetall aus der Gruppe bestehend aus Eisen, Nickel, Kupfer, Kobalt, Mangan, Tantal, Zirkon, Nickel-EisenLegierung und Kobalt-Tantal-Zirkon (CoTaZr)-Legierung ausgewählt ist.

4. Das Verfahren gemäß Anspruch 1, wobei die magnetisierbaren Mikropartikel ein Metalloxid umfassen.

5. Das Verfahren gemäß einem der vorhergehenden Ansprüche, wobei das organische Lösungsmittel aus der Gruppe bestehend aus Toluol, Dimethylbenzol, Tetrahydrofuran und Ethanol ausgewählt ist.

6. Das Verfahren gemäß einem der vorhergehenden Ansprüche, wobei das grenzflächenaktive Mittel Dodecylsulfonsäure-Natriumsalz oder Natriumdodecylbenzolsulfonat ist.

7. Das Verfahren gemäß einem der vorhergehenden Ansprüche, wobei die Konzentration des grenzflächenaktiven Mittels in der organischen Lösung im Bereich von etwa 0,1% (v/v) bis etwa 5% (v/v) liegt.

8. Das Verfahren gemäß einem der vorhergehenden Ansprüche, wobei die Beschichtungsmonomere aus der Gruppe bestehend aus Acrylsäure, Methacrylsäure, Methylmethacrylat, 2-Hydroxylethylmethacrylat, Glykoldimethylacrylat, Ethylenglykoldiacrylat, Ethylenglykoldimethacrylat, Diethylenglykolmethacrylat, Diethylenglykolacrylat, Diethylenglykoldimethacrylat, Diethylenglykoldiacrylat, Triethylenglykoldimethacrylat, Trimethylolpropantrimethacrylat, Pentaerythritittriacrylat, Styrol, Divinylbenzol und einem Gemisch davon ausgewählt sind.

9. Das Verfahren gemäß einem der vorhergehenden Ansprüche, wobei das Funkionalisierungsreagens aus der Gruppe bestehend aus Acrylsäure, Methacrylsäure, Glycidylacrylat, Pentaerythrititdiacrylat und Methacrolein ausgewählt ist.

10. Das Verfahren gemäß einem der vorhergehenden Ansprüche, wobei das Vernetzungsmittel aus der Gruppe bestehend aus Diethylenglykoldimethacrylat, Diethylenglykoldiacrylat, Ethylenglykoldiacrylat, Ethylenglykoldimethacrylat, Trimethylolpropantrimethacrylat, Pentaerythritittriacrylat und Divinylbenzol ausgewählt ist.

11. Das Verfahren gemäß einem der vorhergehenden Ansprüche, wobei der Initiator Benzoylperoxid oder 2,2'-Azobisisobutyronitril ist.

12. Das Verfahren gemäß einem der vorhergehenden Ansprüche, wobei das Verhältnis zwischen der Summe des Kupplungsmittels, der Beschichtungsmonomere, des Funktionalisierungsreagens und des Vernetzungsmittels gegenüber der magnetisierbaren Mikropartikel im Bereich von etwa 1:400 (w/w) bis etwa 1:1 (w/w) liegt.

13. Das Verfahren gemäß einem der vorhergehenden Ansprüche, wobei die Prozentzahlen der Beschichtungsmonomere, des Kupplungsmittels, des Vernetzungsmittels, des Funktionalisierungsreagens und des Initiators in der Summe der Substanzen betragen: etwa 0% bis etwa 80% (v/v) Beschichtungsmonomere, etwa 1% bis etwa 10% (v/v) Kupplungsmittel, etwa 10% bis etwa 80% (v/v) Vernetzungsmittel, etwa 5% bis etwa 40% (v/v) Funktionalisierungsreagens und etwa 1% bis etwa 5% (w/v) Initiator.

14. Beschichtete magnetisierbare Mikropartikel mit aktiven funktionellen Gruppen, welche gemäß dem Verfahren gemäß einem der Ansprüche 1 bis 13 erhältlich sind.

15. Die beschichteten magnetisierbaren Mikropartikel gemäß Anspruch 14, welche einen durchschnittlichen Durchmesser von 10 Nanometer bis 2 Mikrometer aufweisen.

16. Die beschichteten magnetisierbaren Mikropartikel gemäß Anspruch 14 oder 15, welche einen Kern umfassen, der aus der Gruppe bestehend aus Ferritoxid, Kobaltoxid und Nickeloxid ausgewählt ist.

17. Die beschichteten magnetisierbaren Mikropartikel gemäß einem der Ansprüche 14 bis 16, welche weiterhin einen Bindungspartner umfassen, welcher zur Bindung an eine Einheit fähig ist.

18. Die beschichteten magnetisierbaren Mikropartikel gemäß Anspruch 17, wobei der Bindungspartner ein Antikörper oder eine Nukleotidsequenz ist.

19. Die beschichteten magnetisierbaren Mikropartikel gemäß Anspruch 17, wobei der Bindungspartner aus der Gruppe bestehend aus einer Zelle, einer zellularen Organelle, einem Virus, einem Molekül und einem Aggregrat oder Komplex davon ausgewählt ist.

20. Ein Verfahren zur Isolierung einer Einheit, wobei das Verfahren umfasst:
a) Bereitstellen von beschichteten magnetisierbaren Mikropartikeln gemäß einem der Ansprüche 14 bis 19, umfassend einen Bindungspartner, welcher zur Bindung an eine zu isolierende Einheit fähig ist;
b) in Kontakt bringen einer Probe, welche die Einheit enthält oder enthalten soll, mit den in Schritt a) bereitgestellten beschichteten magnetisierbaren Mikropartikeln unter Bedingungen, die eine Bindung zwischen der Einheit und dem Bindungspartner zulassen; und
c) Gewinnung der beschichteten magnetisierbaren Mikropartikel aus der Probe mit magnetischer Kraft.

21. Das Verfahren gemäß Anspruch 20, weiterhin umfassend Gewinnung der Einheit aus den beschichteten magnetisierbaren Mikropartikeln.

22. Das Verfahren gemäß Anspruch 20 oder 21, wobei die Isolierung auf einem Chip durchgeführt wird.

23. Ein Verfahren zur Manipulation einer Einheit, wobei das Verfahren umfasst:
a) Bereitstellen von beschichteten magnetisierbaren Mikropartikeln gemäß einem der Ansprüche 14 bis 19, umfassend einen Bindungspartner, welcher zur Bindung an eine zu manipulierende Einheit fähig ist;
b) Kuppeln der Einheit an die in Schritt a) bereitgestellten beschichteten magnetisierbaren Mikropartikel über eine Bindung zwischen der Einheit und dem Bindungspartner, um einen Einheit-beschichtete-magnetisierbare-Mikropartikel-Komplex zu bilden; und
c) Manipulieren des Einheit-beschichtete-magnetisierbare-Mikropartikel-Komplexes mit einer magnetischen Kraft,
wodurch die Einheit manipuliert wird.

24. Das Verfahren gemäß Anspruch 23, weiterhin umfassend die Gewinnung der Einheit aus den beschichteten magnetisierbaren Mikropartikeln.

25. Das Verfahren gemäß Anspruch 23 oder 24, wobei die Manipulation auf einem Chip durchgeführt wird.

26. Das Verfahren gemäß einem der Ansprüche 23 bis 25, wobei die Manipulation aus der Gruppe bestehend aus Transport, Fokussierung, Anreicherung, Konzentration, Aggregation, Einfangen, Abstoßung, Schweben, Abtrennung, Fraktionierung, Isolierung und linearer Bewegung oder anders gerichteter Bewegung der Einheit ausgewählt ist.

## Revendications

1. Procédé de production de microparticules magnétisables revêtues ayant des groupes fonctionnels actifs, lequel procédé comprend :
a) la dispersion de microparticules magnétisables d'un diamètre variant de 5 à 1000 nanomètres dans un solvant organique contenant un tensioactif ;
b) l'ajout d' un agent de couplage, de monomères de revêtement, d'un réactif de fonctionnalisation, d'un agent de réticulation et d'un initiateur audit solvant organique contenant lesdites microparticules magnétisables dispersées, pour permettre l'attachement dudit agent de couplage à la surface desdites microparticules magnétisables et la dispersion desdits monomères de revêtement de manière homogène sur la surface desdites microparticules magnétisables avec ledit agent de couplage attaché ;
c) l'initiation et l'achèvement de la polymérisation desdits monomères de revêtement pour former des microparticules magnétisables revêtues ayant des groupes fonctionnels actifs en l'absence d'oxygène, où des polymères desdits monomères de revêtement s'attachent à la surface desdites microparticules magnétisables revêtues via ledit agent de couplage, une multiplicité desdits polymères sont réticulés ensemble par ledit agent de réticulation et ledit réactif de fonctionnalisation est lié auxdits polymères, agent de réticulation et/ou agent de couplage, de façon qu'au moins un groupe fonctionnel dudit réactif de fonctionnalisation reste disponible,
dans lequel le réactif de couplage est choisi dans le groupe constitué par le bis(2-hydroxyéthylméthacrylate)phosphate, le bis(triméthylolpropanediacrylate)phosphate et le bis(pentaérythritoltriacrylate)phosphate.

2. Procédé selon la revendication 1, dans lequel les microparticules magnétisables comprennent une composition à base d'un métal de transition ou alliage de celui-ci.

3. Procédé selon la revendication 2, dans lequel le métal de transition est choisi dans le groupe constitué par le fer, le nickel, le cuivre, le cobalt, le manganèse, le tantale, le zirconium, un alliage nickel-fer et un alliage cobalt-tantale-zirconium (CoTaZr).

4. Procédé selon la revendication 1, dans lequel les microparticules magnétisables comprennent un oxyde de métal.

5. Procédé selon l'une quelconque des revendications précédentes, dans lequel le solvant organique est choisi dans le groupe constitué par le toluène, le diméthylbenzène, le tétrahydrofuranne et l'éthanol.

6. Procédé selon l'une quelconque des revendications précédentes, dans lequel le tensioactif est le sel de sodium de l'acide dodécylsulfonique ou le dodécylbenzènesulfonate de sodium.

7. Procédé selon l'une quelconque des revendications précédentes, dans lequel la concentration du tensioactif dans la solution organique varie d'environ 0,1 % (v/v) à environ 5 % (v/v).

8. Procédé selon l'une quelconque des revendications précédentes, dans lequel les monomères de revêtement sont choisis dans le groupe constitué par l'acide acrylique, l'acide méthacrylique, le méthacrylate de méthyle, le méthacrylate de 2-hydroxyéthyle, le diméthylacrylate de glycol, le diacrylate d'éthylène glycol, le diméthacrylate d'éthylène glycol, le méthacrylate de diéthylène glycol, l'acrylate de diéthylène glycol, le diméthacrylate de diéthylène glycol, le diacrylate de diéthylène glycol, le diméthacrylate de triéthylène glycol, le triméthacrylate de triméthylolpropane, le triacrylate de pentaérythritol, le styrène, le divinylbenzène et un mélange des ceux-ci.

9. Procédé selon l'une quelconque des revendications précédentes, dans lequel le réactif de fonctionnalisation est choisi dans le groupe constitué par l'acide acrylique, l'acide méthacrylique, l'acrylate de glycidyle, le diacrylate de pentaérythritol et la méthacroléine.

10. Procédé selon l'une quelconque des revendications précédentes, dans lequel l'agent de réticulation est choisi dans le groupe constitué par le diméthacrylate de diéthylène glycol, le diacrylate de diéthylène glycol, le diacrylate d'éthylène glycol, le diméthacrylate d'éthylène glycol, le triméthacrylate de triméthylolpropane, le triacrylate de pentaérythritol et le divinylbenzène.

11. Procédé selon l'une quelconque des revendications précédentes, dans lequel l'initiateur est le peroxyde de benzoyle ou le 2,2'-azobisisobutyronitrile.

12. Procédé selon l'une quelconque des revendications précédentes, dans lequel le rapport entre la somme de l'agent de couplage, des monomères de revêtement, du réactif de fonctionnalisation et de l'agent de réticulation contre les microparticules magnétisables varie d'environ 1:400 (p/p) à environ 1:1 (p/p).

13. Procédé selon l'une quelconque des revendications précédentes, dans lequel les pourcentages de monomères de revêtement, agent de couplage, agent de réticulation, réactif de fonctionnalisation et initiateur dans la somme des substances sont les suivants : d'environ 0 % à environ 80 % (v/v) de monomères de revêtement, d'environ 1 % à environ 10 % (v/v) d'agent de couplage, d'environ 10 % à environ 80 % (v/v) d'agent de réticulation, d'environ 5 % à environ 40 % (v/v) de réactif de fonctionnalisation et d'environ 1 % à environ 5 % (p/v) d'initiateur.

14. Microparticules magnétisables revêtues ayant des groupes fonctionnels actifs, qui sont susceptibles d'être obtenues selon le procédé de l'une quelconque des revendications 1 à 13.

15. Microparticules magnétisables revêtues selon la revendication 14, qui ont un diamètre moyen de 10 nanomètres à 2 micromètres.

16. Microparticules magnétisables revêtues selon les revendications 14 ou 15, qui comprennent un noyau choisi dans le groupe constitué par l'oxyde de ferrite, l'oxyde de cobalt et l'oxyde de nickel.

17. Microparticules magnétisables revêtues selon l'une quelconque des revendications 14 à 16, qui comprennent en outre un partenaire de liaison qui est capable de se lier à un fragment.

18. Microparticules magnétisables revêtues selon la revendication 17, dans lesquelles le partenaire de liaison est un anticorps ou une séquence de nucléotides.

19. Microparticules magnétisables revêtues selon la revendication 17, dans lesquelles le partenaire de liaison est choisi dans le groupe constitué par une cellule, une organelle cellulaire, un virus, une molécule et un agrégat ou complexe de ceux-ci.

20. Procédé d'isolement d'un fragment, lequel procédé comprend :
a) la fourniture de microparticules magnétisables revêtues selon l'une quelconque des revendications 14 à 19 comprenant un partenaire de liaison qui est capable de se lier à un fragment qui doit être isolé ;
b) la mise en contact d'un échantillon contenant ou suspecté de contenir ledit fragment avec lesdites-microparticules magnétisables revêtues fournies à l'étape a) dans des conditions permettant la liaison entre ledit fragment et ledit partenaire de liaison ; et
c) la récupération desdites microparticules magnétisables revêtues à partir dudit échantillon avec une force magnétique.

21. Procédé selon la revendication 20, comprenant en outre la récupération du fragment à partir des microparticules magnétisables revêtues.

22. Procédé selon la revendication 20 ou 21, dans lequel l'isolement est mis en oeuvre sur une puce.

23. Procédé de manipulation d'un fragment, lequel procédé comprend :
a) la fourniture de microparticules magnétisables revêtues selon l'une quelconque des revendications 14 à 19 comprenant un partenaire de liaison qui est capable de se lier à un fragment qui doit être manipulé ;
b) le couplage dudit fragment auxdites microparticules magnétisables revêtues fournies à l'étape a) par liaison entre ledit fragment et ledit partenaire de liaison pour former un complexe fragment-microparticules magnétisables revêtues ; et
c) la manipulation dudit complexe fragment-microparticules magnétisables revêtues avec une force magnétique,
ledit fragment étant ainsi manipulé.

24. Procédé selon la revendication 23, comprenant en outre la récupération du fragment à partir des microparticules magnétisables revêtues.

25. Procédé selon la revendication 23 ou 24, dans lequel la manipulation est mise en ouvre sur une puce.

26. Procédé selon l'une quelconque des revendications 23 à 25, dans lequel la manipulation est choisie dans le groupe constitué par le transport, la focalisation, l'enrichissement, la concentration, l'agrégation, le piégeage, la répulsion, la lévitation, la séparation, le fractionnement, l'isolement et le déplacement linéaire ou dans une autre direction du fragment.
